(19) Europäisches Patentamt
European Patent Office
Office européen des brevets

(11) EP 2 137 192 B1

(12) **EUROPEAN PATENT SPECIFICATION**

(45) Date of publication and mention of the grant of the patent:
**19.02.2014 Bulletin 2014/08**

(21) Application number: **08735211.8**

(22) Date of filing: **14.04.2008**

(51) Int Cl.:
***C07D 491/10*** *(2006.01)* ***A61K 31/4355*** *(2006.01)*
***A61P 25/00*** *(2006.01)*

(86) International application number:
**PCT/EP2008/002929**

(87) International publication number:
**WO 2009/127218 (22.10.2009 Gazette 2009/43)**

(54) **Derivatives of galantamine as pro-drugs for the treatment of human brain diseases**

Galantaminderivate als Prodrug-Verbindungen zur Behandlung humaner Hirnerkrankungen

Dérivés de galantamine comme promédicaments pour le traitement de maladies du cerveau humain

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**30.12.2009 Bulletin 2009/53**

(73) Proprietor: **Neurodyn Life Sciences Inc.**
**Charlottetown, P.E.I., CIA 4P3 (US)**

(72) Inventor: **MAELICKE, Alfred**
**55268 Nieder-Olm (DE)**

(74) Representative: **Boeckh, Tobias et al**
**Hertin und Partner**
**Rechts- und Patentanwälte**
**Kurfürstendamm 54/55**
**10707 Berlin (DE)**

(56) References cited:
**EP-A- 1 777 222** **WO-A-99/21561**

- **HAN S-Y ET AL: "SYNTHESIS AND BIOLOGICAL ACTIVITY OF GALANTHAMINE DERIVATIVES AS ACETYLCHOLINESTERASE (ACHE) INHIBITORS" BIOORGANIC & MEDICINAL CHEMISTRY LETTERS, PERGAMON, ELSEVIER SCIENCE, GB, vol. 1, no. 11, 1 January 1991 (1991-01-01), page 579/580, XP001181208 ISSN: 0960-894X**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a selected galantamine derivative for use as a medicament, having in comparison to galantamine higher efficacy and lower levels of adverse side effects in treatment of human brain diseases.

The plant alkaloid galantamine has been described as a cholinesterase inhibitor (ChE-I) and as a nicotinic acetylcholine receptor (nAChR) sensitizing agent (APL; allosterically potentiating ligand), and galantamine has been proposed for the treatment of several human brain diseases, including Alzheimer's disease (AD). Presently, the compliance of Alzheimer patients to treatment with ChE-I and APL is rather low, of the order of 20%, a key reason being the adverse effects nausea, diarrhea, vomiting, anorexia and muscle cramps. In the case of galantamine, the majority of these adverse effects is due to actions of the drug while passing through the gastro-intestinal tract, and to its rather limited permeation through the blood-brain barrier (BBB) into the brain. To help patients coping with the adverse effects of galantamine, the manufacturer's recommended daily dose of the drug is limited to 16-24 mg per day, and this dose is slowly reached by stepwise dose increase, beginning at 4 mg/day and over a period of 2-3 months.

The rather low levels of accumulation of galantamine in the brain, when administered as the unmodified drug, are a serious disadvantage with respect to the drug's therapeutic use, i.e. for the treatment of cognitive disorders, such as AD. As indicated by the brain-to-plasma ratio of ~ 1.3, only a small part of the administered drug reaches the brain, and the high levels of the drug in other (peripheral) tissues cause most, if not all, of the observed adverse effects. The mostly peripheral action of galantamine is also indicated in its previous use for the treatment of a number of neuromuscular disorders, including Myasthenia gravis and poliomyelitis.

In WO2007/039138 and EP 1777222 reference is made to the low hydrophobicity and related limited partition into the human brain of galantamine, and several procedures for overcoming these drawbacks of a medication that is supposed to act on target molecules located in the brain's central nervous system are proposed. In the same document numerous derivatives of galantamine that significantly improve transport of the respective compound through the blood-brain barrier (BBB) are described and they are proposed as drugs for the treatment of a variety of diseases associated with cognitive deficits.

EP-A 648 771, EP-A 649 846 and EP-A 653 427 all describe galantamine derivatives, a process for their preparation and their use as medicaments, however none of these applications considers ways and means of enhancing penetration through the blood-brain barrier and brain-to-plasma ratio of base compounds and derivatives.

US 6,150,354 refers to several galantamine analogues for the treatment of Alzheimer's disease. However, selective chemical modification for the purpose of increasing penetration through the blood-brain barrier is not considered.

WO 01/74820, WO 00/32199 and WO 2005030333 refer to derivatives and analogues of galantamine for the treatment of a variety of human brain and other diseases, and acute functional brain damage. However, selective chemical modifications or other means of improving blood-brain barrier penetration and brain-to-blood concentration ratio are not considered.

WO 88/08708, WO 99/21561, WO 01/43697 and US 2003/0162770 refer to derivatives and analogues of galantamine for the treatment of various cognitive symptoms. However, selective chemical modifications or other means of improving brain-to-blood concentration ratio are not considered.

WO 2005/030713 refers to a method for the synthesis of optical isomers of galantamine from a narwedine bromoamide derivative. However, it does not deal with other derivatives of galantamine, or their use as medicaments, or chemical modifications aimed at enhancing brain-to-blood concentration ratio of said compounds.

WO 97/40049 describes several derivatives of benzazepines and related compounds that may be applied for the treatment of Alzheimer's disease. However, no concept is provided in this application for increasing the penetration of compounds through the blood-brain barrier and providing high brain-to-blood concentration ratio.

The object of the present invention is to provide compounds usable as pro-drugs or as a medicament having high pharmacodynamic effects in the brain's central nervous system and low peripheral side effects.

This object is met by the compound and composition as provided in the claims.

The proposed derivatives were designed as pro-drugs, in the sense that they are able to effectively pass the blood brain barrier (BBB) and, after passing the BBB, they are substrates of endogenous enzymes and, upon enzymatic cleavage, produce galantamine. As a result of enzymatic cleavage to galantamine of such pro-galantamines in the brain, a significantly higher local concentration of galantamine is achieved in the brain than by administration of the same dose of original galantamine. The relatively higher drug concentration in the brain achieved by pro-galantamine administration will then result in higher efficacy at a given dose, and the better brain-to-peripheral tissues distribution will result in fewer or less significant side effects of treatment. These effects are significant improvements of present treatment regimens because the efficacy as treatment for brain diseases of unmodified galantamine (and all other ChE-I presently approved for this purpose) is rather limited, albeit statistically significant, possibly due to low dosing. Thus, efficacy is usually reached only after careful (months-long) up-titration of daily dose, so as to maintain sufficient compliance of patients to the largely gastro-intestinal side effects associated with ChE-I treatment.

It was found by the inventors that careful selection concerning the type of substituent and the position of

substitution using galantamine as base structure result in highly efficacious compounds. Such very efficacious compounds having good blood brain barrier passing properties and being efficiently cleaved by an esterase after passage through the BBB are obtained with compounds having the general formula I

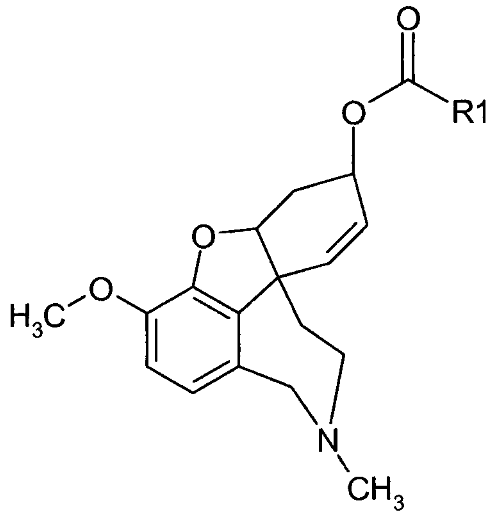

with R1 being a substituent having particular sterical and hydrophobic properties.

The present invention therefore relates to an ester of galantamine that as such has little or no activity as ChE-I and APL compared to galantamine. Therefore, as long as the compound remains uncleaved, it does not interact with the usual target molecules of galantamine and hence is largely inactive in producing therapeutic and/or side effects.

The reduced reactivity of pro-galantamines is demonstrated by the following results:

1. Significantly reduced activity as ChE-I, as compared to galantamine.

2. Reduced activity as nicotinic APL, as compared to galantamine.

3. Reduced gastro-intestinal side effects, as compared to galantamine.

All these approaches were investigated and are explained in the examples and shown in the figures.

According to the invention it has been discovered that a particular ester of galantamine displays unexpectedly high brain-to-blood concentration ratios ($R_{BB}$-proGal > 6, as compared to $R_{BB}$-Gal ~ 1.3) and in the brain it is relatively slowly enzymatically cleaved to galantamine. Therefore, as is discussed below in more detail, the pro-galantamine is exceptionally well suited for the treatment of human diseases associated with cholinergic deficits, such as Alzheimer's disease, Parkinson's disease, Schizophrenia and a variety of other psychiatric disorders.

The esters of galantamine to which the present invention refers to have the following general structure:

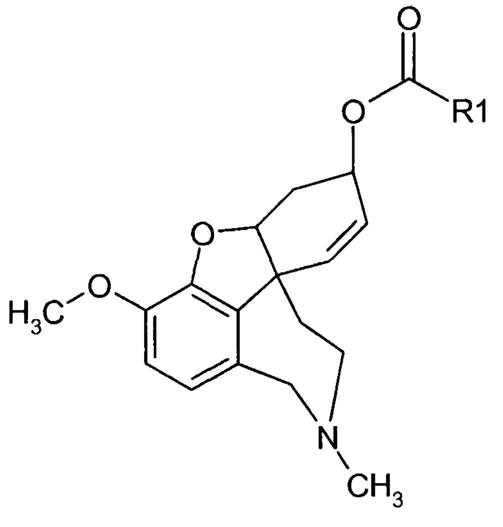

Formula I

wherein R1 either is $CH(C_2H_5)CH_3$, $CH_2$-$C(CH_3)_3$, or cyclopropane or being an optionally substituted aromatic or hetero-aromatic 5- or 6-membered ring. Specifically, such aromatic and hetero-aromatic rings include benzene, naphthalene, thiophene, pyrrole, imidazole, pyrazole, oxazole and thiazole, in case that they are used as medicaments or pro-drugs for the treatment of neurodegenerative or psychiatric or neurological disease associated with a cholinergic deficit.

According to claim 1 the invention relates specifically to an ester of galantamine with the following structure:

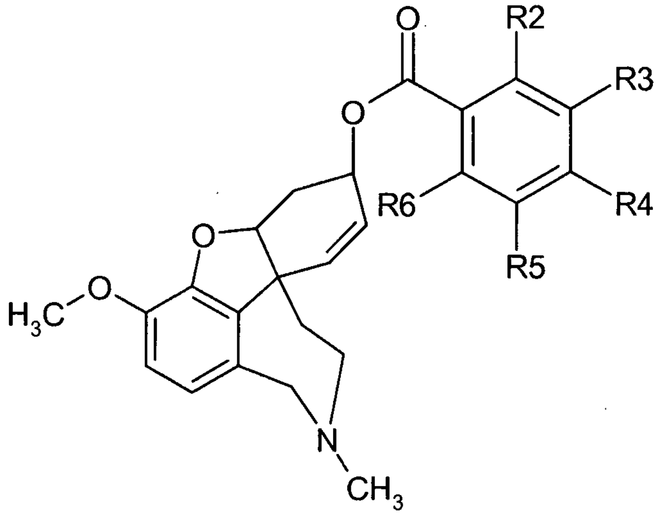

Formula II

wherein R2-R6 comprising any substituent selected from H, halogen, optionally substituted $C_1$-$C_3$ alkyl or cyclopropyl, OH, O-alkyl, SH, S-alkyl, $NH_2$, NH-alkyl, N-dialkyl, optionally substituted aryl or hetero-aryl, whereby neighbouring substitutents can cooperate to form an additional ring.

Herein the term "pro-drug" refers to a derivative of galantamine (base compound) wherein the group(s) added or replaced on said base compound are cleaved or returned to the hydroxyl group originally contained in the base compound, when the derivative has reached the area or site of action. Thus, in case of a "pro-drug", an effective agent is administrated as a derivative (which is said pro-drug), however, the compound mainly or exclusively effective at the target site within the brain is the agent itself, not the derivatised compound or metabolites other than the base compound thereof.

The term "derivative" refers to any change of a base compound defined in the present application. The term "derivative" is used to describe a compound which either can be a pro-drug, or can be an effective agent itself/in its own right or in the derivatised form.

The term "pro-galantamine" is used for any derivative of galantamine described herein which can be cleaved by an enzyme (esterase) resulting in galantamine.

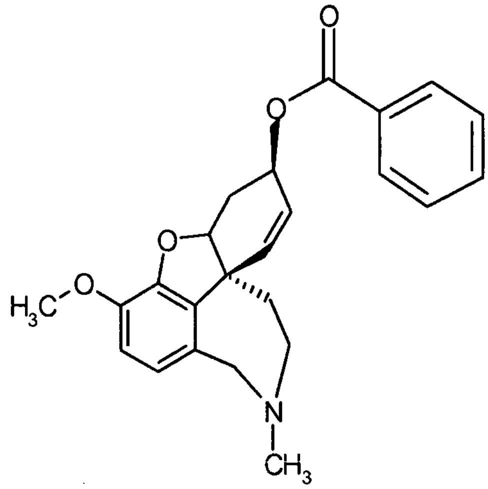

for use as a medicament or pro-drug for the treatment of neurodegenerative or psychiatric or neurological disease associated with a cholinergic deficit, wherein the disease is selected from Alzheimer's and Parkinson's disease, other types of dementia, schizophrenia, epilepsy, neuritis, various types of poisoning, anesthesia, particularly neuroleptic anesthesia, autism, spinal cord disorders, inflammation, particularly central inflammatory disorders, postoperative delirium and/or subsyndromal postoperative delirium, neuropathic pain, subsequences of the abuse of alcohol and drugs, addictive alcohol and nicotine craving, and subsequences of radiotherapy.

The above mentioned compound was not yet described for the treatment of such diseases.

Furthermore, compounds of formula I having aromatic or hetero-aromatic 5- or 6-membered ring, selected from substituted benzene with the proviso that it is not 2-fluorobenzene or 3-nitro-4-fluorobenzene, optionally substituted naphthalene, thiophene, pyrrole, imidazole, pyrazole, oxazole, thiazole; or $CH(C_2H_5)CH_3$, $CH_2$-$C(CH_3)_3$, or cyclopropane are according to the knowledge of the inventor not yet described at all.

The compounds disclosed in the present application are selected from

The terms "sensitising agent" and "allosterically potentiating ligand, APL" refer to effectors that enhance cholinergic neurotransmission by interaction with an allosteric site at cholinergic receptors.

The terms "cholinergic enhancer" and "cholinergic agent" refer to compounds that enhance/modulate cholinergic neurotransmission by inhibition of cholinesterases, by allosteric sensitisation and/or direct activation of cholinergic receptors and/or by activating/modulating relevant intracellular pathways via second messenger cascades.

A derivative or pro-drug has an "enhanced blood-brain barrier permeability "according to the present invention or an "enhanced blood-brain barrier penetration" if, after administration of a pro-drug or derivative thereof to a living organism, a higher amount of said compound penetrates through the BBB of that organism.

A compound of the present invention provides an increased "brain-to-blood concentration ratio" or "brain-to-tissue concentration ratio" resulting in a higher level of effective agent in the brain, as compared to administration of the base compound without derivatisation. Methods for determination of an enhanced BBB permeability are disclosed in WO 2007/039138.

The "base compound" as well as the "effective agent" according to the present invention is galantamine. The effective agent is obtained by (local) enzymatic cleavage of the derivative.

"logP" is defined as the decadic logarithm of the partition coefficient P which is the ratio of the concentration of a compound in aqueous phase to the concentration of a compound in immiscible solvent, as the neutral molecule.

The term "alkyl" shall mean a straight, branched or cyclic alkyl group. As "alkyl" $C_1$ to $C_{10}$ alkyl groups are preferred, $C_2$ to Cg groups are more preferred and $C_2$ to $C_6$ groups are most preferred. $C_1$ to $C_{10}$ means alkyl groups of the stated number of carbon atoms. Examples include, but are not limited to methyl, ethyl, n-propyl, iso-propyl, n-butyl, isobutyl, sec-butyl, t-butyl, and straight and branched chain pentyl, hexyl, heptyl, octyl, nonyl and decyl etc...or the according cyclic alkyls.

The term "halo" shall mean chloro, fluoro, bromo and iodo.

The term "aryl" shall mean phenyl having 0, 1, 2, 3, 4 or 5 substituents independently selected from the group of alkyl, alkoxy, alkylcarbonyl, halo- or trihalomethyl.

The term "cycloalkyl" shall mean a cycloalkyl group of from 3 to 10 carbon atoms and including multiple ring alkyls such as for example, adamantyl, camphoryl; and 3-noradamantyl.

In any case when a range between two limits is described it is meant that any value or integer in this range is disclosed. For example "$C_1$-$C_{10}$" means $C_1$, $C_2$, $C_3$, $C_4$, $C_5$, $C_6$ ,$C_7$, $C_8$, $C_9$ or $C_{10}$; or "between 0,1 and 1" means 0,1, 0,2, 0,3, 0,4, 0,5, 0,6, 0,7, 0,8, 0,9 or 1.

The stereo chemistry of the described derivatives are the same as that of galantamine.

Benzoyl esters of galantamine were previously described in WO 9921561 A1 Davis, Bonnie M. for a method of treatment of disorders of attention with galanthamine, lycoramine, and related compounds, but no syntheses or analytical or other data were provided for these compounds.

Substituted benzoyl esters were previously described in "Synthesis and biological activity of galanthamine derivatives as acetylcholinesterase (AChE) inhibitors" by Han, So Yeop; Mayer, Scott C.; Schweiger, Edwin J.; Davis, Bonnie M.; Joullie, Madeleine M. Dep. Chem., Univ. Pennsylvania, Philadelphia, PA, USA. Bioorganic & Medicinal Chemistry Letters (1991), 1(11), 579-80. CODEN: BMCLE8 ISSN: 0960-894X. Journal written in English. CAN 116: 83569 AN 1992:83569 CAPLUS. In this document the synthesis of several ester and carbamate derivatives of galanthamine are described as well as it was suggested that these compounds are potential therapeutic agents in the treatment of Alzheimer's disease, based on their properties as AChE inhibitors.

In contrast to the teaching of these documents, the galantamine ester of the present invention has little, if any, activity as acetylcholinesterase inhibitor but rather is the substrate of said enzyme (see above).

As representatively demonstrated for the benzoyl derivative in Figure 1, the ester of the present invention has little, if any cholinesterase inhibitory activity but rather is hydrolysed by cholinesterases to form galantamine and accordingly act as pro-drug of galantamine. As soon as galantamine is generated from the compound, it acts as ChE-I and APL, as has previously been described. The structures of the tested derivative can be seen in table A. As a comparative derivative a non-cleavable galantamine ether is also tested. Such derivative results in negative values of inhibition. In derivative Gln 1063 R1 in formula I is $-O-Si(CH_3)_2-C(CH_3)_2-C(CH_3)_2H$.

Rather than inhibiting cholinesterases, the pro-galantamine referred to in the present document is a substrate of the enzyme, as is exemplarily demonstrated in Figure 2.

The data of figures 1 and 2 demonstrate that the pro-galantamine of the present invention does not act as efficient inhibitor of cholinesterase, as was described in the earlier documents discussed above. Instead, it is a substrate of these enzymes. Similarly, it also does not interact to the same extent as galantamine with neuronal nicotinic acetylcholine receptors (figure 3).

The pro-galantamine of the present invention therefore either does not interact, or only to a very limited extend, with the established target molecules of galantamine, in particular cholinesterases and neuronal nicotinic acetylcholine receptors. As pro-drug it therefore has rather limited, if any, efficacy as cognition enhancer, and also produces only limited peripheral and central side effects, as compared to galantamine (see further below).

As is exemplified and demonstrated by pharmacokinetics in mice (figures 4a and 4b, table 1), R1-benzoyl-galantamine displays an unexpectedly high brain-to-blood concentration ratio ($R_{BB}$-proGal > 19), a large initial concentration in the brain, and it is only slowly cleaved to galantamine, as seen in the delayed appearance of a galantamine peak in brain and blood. The $R_{BB}$-value is significantly larger than what was expected from the logP value which probably is due to the slow cleavage of the pro-drug in the brain and a depot effect thereby produced.

In table 1, the key pharmacokinetic data of benzoyl-galantamine, of several other R1-pro-galantamines and of galantamine (for comparison) are listed.

Table 1: Pharmacokinetic data of several R1-pro-galantamines in the mouse

| Gln number (for reference see table A) | logP | Co (Brain) | R-Pro | R-Gal |
|---|---|---|---|---|
| 1062 | 3.0 | 4812 | 19.3 | 2.2 |
| 1067 | 2.8 | 4665 | 7.5 | 1.7 |
| 0979 | 2.5 | 3166 | 6.2 | 2.4 |
| 0993 | 3.7 | 2150 | 6.4 | 0.6 |
| 0978 | 2.2 | 1985 | 6.9 | 2.3 |
| 1076 | 2.4 | 1245 | 1.1 | 1.0 |
| Gal | 1.7 | 1741 | | 1.2 |

Co is the highest pro-galantamine concentration (ng/ml) achieved in mouse brain after i.v. injection of 3 mg/kg of pro-galantamine. R-Pro ist he brain-to-blood concentration ratio of pro-galantamine, R-Gal that of galantamine under these experimental conditions. For comparison, Co and R-Gal are also provided for i.v. injection of the same amount of galantamine.

These data establish that only a particular selection of R1 substitutions is capable of producing the following advantageous properties of pro-galantamines; high initial concentration in the brain, large $R_{BB}$, and slow enzymatic conversion to galantamine. In addition (not shown in the table), the preferred R1-prodrugs display little, if any side effects, as they are only very slowly converted to galantamine, thereby largely protecting them from acting as galantamine while being transported from the site of administration to the sites of action in the brain.

These properties may have significant impact for the use of these compounds as drugs in Alzheimer's disease and other brain diseases. As is representatively shown in figure 5, R1-pro-galantamines of the present invention display in ferrets much less gastro-intestinal side effects than galantamine. Ferrets were used in these studies as they are known to be particularly sensitive to gastro-intestinal side effects. In addition to the classical emetic responses to galantamine and other ChE inhibitors, we recorded salivation (SA), shivering (SH), respiratory problems (RP) and diarrhea (DI) at the levels "none; 0", "moderate; 0.5" (behaviour observed at low frequency and/or at low intensity) and "intense; 1.0" (behaviour observed frequently and/or continuously and/or at high intensity), and summated the scores for the four animals each used per drug dose in these studies.

The data depicted in figure 5 for the two pro-galantamines demonstrate that their side effects profile in ferrets is much less severe (5-6 times less) than that of the same dose of galantamine. The advantageous side effects profile is probably due to the reduced affinity of interaction of these R1-pro-galantamines with cholinesterases and neuronal nicotinic acetylcholine receptors (figures 1, 3).

The advantages of enhanced transport of selected R1-produgs through the blood-brain barrier into the brain, enzymatic conversion to galantamine close to target sites in the central nervous system, and interaction with such sites is producing enhanced reversal of drug-induced amnesia in mice, as is shown in figure 6 for three pro-glantamines (and for galantamine in comparison).

The data of figure suggest that Gln-1062 is approximately 4-times more efficacious than galantamine in reversing scopolamine-induced amnesia in mice. It may be expected that a similar or larger increase in drug efficacy can be achieved in man when the particular R1-pro-galantamine is administered instead of galantamine. The advantageous drug properties of R1-pro-galantamines (higher efficacy, lesser or less intense side effects) were also shown in other animal models.

In summary, the compound of this invention is particularly useful as a medicament for the treatment of human brain diseases associated with a cholinergic deficit, including the neurodegenerative diseases Alzheimer's and Parkinson's disease and the neurological/psychiatric diseases vascular dementia, schizophrenia and epilepsy. Based on preclinical studies using various animal models, the claimed compound has dramatically reduced side effects as compared to galantamine, including much fewer, if any, incidents of emetic responses, diarrhea and vomiting. Moreover, when enzymatically cleaved, the resulting galantamine displays an advantageous pharmacokinetic profile in the brain and, due to its enhanced concentration level in the brain, displays also enhanced efficacy in interaction with the target molecules located in the brain. Taken together, these properties make the administration of galantamine as a R1-prodrug a preferred medication in the diseases mentioned above.

Figures

Figure 1: Brain esterase inhibition by galantamine and several pro-galantamines.
A 20% mouse brain homogenate was used, supplemented with 200 μM of acetylthiocholine as substrate, and the initial reaction kinetics were measured according to Riddles PW, Blakeley RL, Zerner B., "Reassessment of Ellman's reagent." Methods Enzymol. 1983;91:49-60. As shown in the figure, even 50 μM of the respective pro-galantamines were unable to achieve a level of inhibition of brain cholinesterase that is comparable in size to that of 1 μM galantamine. A non-cleavable galantamine derivative (Gln 1063) leads to negative values. In derivative Gln 1063 R1 in formula I is $-O-Si(CH_3)_2-C(CH_3)_2-C(CH_3)_2H$.

Figure 2: Enzymatic cleavage of pro-galantamine to galantamine.
Butyrylcholinesterase, 25 units/ml, was used. Reaction temperature was 37°C. Appearance of the fluorescent reaction product galantamine was determined by fluorescence detection.

Figure 3: Interaction of galantamine and pro-galantamine with a4ß2 neuronal nicotinic acetylcholine receptor ectopically expressed in HEK-293 cells.
The increase in response to acetylcholine in the presence of galantamine and Gln-1062, respectively, was determined by whole-cell patch clamp recording. Galantamine achieved a maximal enhancement of response of ~ 40% whereas the pro-galantamine achieved a maximal enhancement of only ~ 17%.

Figure 4: Pharmacokinetics of pro-galantamine Gln-1062 (3mg/kg) in the mouse
Figure 4a shows the measurable concentration of applied pro-galantamine and the resulting concentration of galantamine by cleavage of the pro-galantamine in brain and brain. The curve starting with the highest concentration refers to derivative GLN-1062, which is benzoyl-galantamine, in brain. Figure 4b is an excerpt ("zoom") of Figure 4a, showing the concentration range between 0.00 and 1.00 μg/g (substance/body weight) more in detail. In Figure

4b the curve above refers to the concentration of resulting galantamine in brain, the middle curve shows the concentration of galantamine in blood and the curve starting with a concentration of about 0.3 μg/g and decreasing refers to the concentration of GLN-1062 in blood.

Figure 5: Behavioural index for gastro-intestinal side effects in ferrets following application of galantamine and several R1-pro-galantamines, respectively.

Figure 6: Reversal from scopolamine-induced amnesia in mice, in the presence of galantamine and several R1-pro-galantamines, respectively.
Scopolamine induces a memory deficit that can be measured by increased alternation in a T-maze trial. The cognition enhancing drugs were i.p. injected at several different doses together with scopolamine 20 min before a T-maze trial. Recovery was measured as a function of dose, and the $EC_{50}$ was determined for each drug.

<u>Examples</u>

<u>Examples of chemical synthesis and chemical properties of derivatives:</u>

Abbreviations: DCM: dichloromethane; DMAP: 4-dimethylaminopyridine; DCC: dicyclohexylcarbodiimide; DCHU: dicyclohexylurea

**<u>General procedure 1</u>**

To the solution of (-)-galanthamine hydrobromide (1.0 mole) and triethyl amine (4.0 mol) in DCM (30 mL), DMAP (0.5 mol) was added followed by respective acid chloride or acid anhydride (1.2 mol). The mixture was stirred overnight at room temperature under argon. The reaction mixture was washed with 10% $NaHCO_3$ and brine, dried ($Na_2SO_4$) and concentrated. The crude compound obtained was purified by column chromatography or recrystallization to give the pure product.

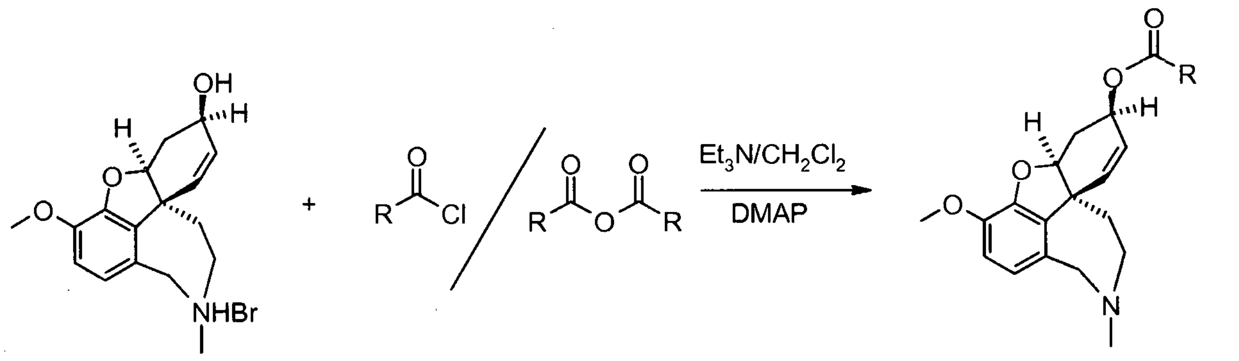


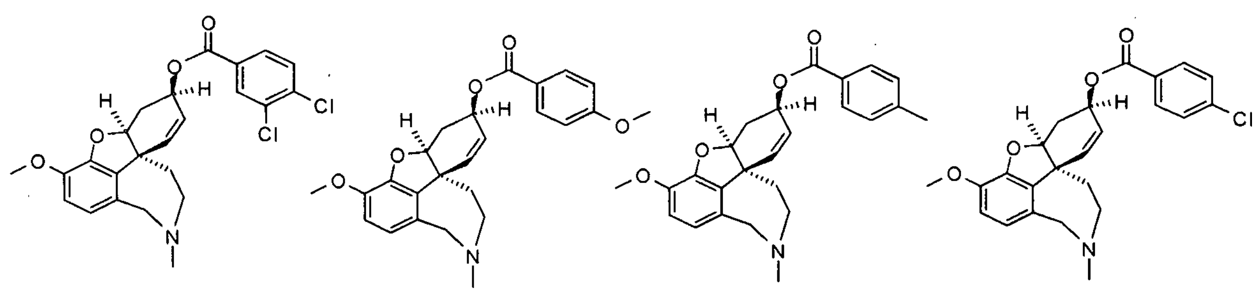

Using this procedure the following compounds were obtained:

**Example 1**: O-Benzoyl-galanthamine (=(4aS,6R,8aS)-4a,5,9,10,11,12-Hexahydro-3-methoxy-11-methyl-6H-benzofuro [3a,3,2-ef][2]benzazepin-6-ol, benzoate (ester)); yield: 78%

**Example 2:** O-3,4-Dichlorobenzoyl-galanthamine (=(4aS,6R,8aS)-4a,5,9,10,11,12-Hexahydro-3-methoxy-11-methyl-6H-benzofuro[3a,3,2-ef][2]benzazepin-6-ol, 3,4-dichlorobenzoate (ester)); off-white solid; mp. 69-70°C.

$^1H$ NMR (200 MHz, $CDCl_3$) $\delta$ (ppm) 8.02 (d, *J* = 1.88 Hz, 1H), 7.81 (dd, *J* = 1.88 Hz, *J* = 8.38 Hz, 1H), 7.38 (d, *J* = 8.32 Hz, 1H), 6.62 (d, *J* = 8.18 Hz, 1H), 6.52 (d, *J* = 8.18 Hz, 1H), 6.32 (d, *J* = 10.34 Hz, 1H), 5.89-5.97 (m, 1H), 5.51 (t, *J* = 4.43 Hz, 1H), 4.58 (s, 1H), 4.07 (d, *J* = 15.16 Hz, 1H), 3.18 (s, 3H), 3.61 (d, *J* = 15.16 Hz, 1H), 3.21-3.45 (m, 1H), 2.96-3.05 (m, 1H), 2.66-2.76 (m, 1H), 2.34 (s, 3H), 2.0-2.19 (m, 2H), 1.51-1.59 (m, 1H).

**Example 3:** O-4-Methoxybenzoyl-galanthamine (=(4aS,6R,8aS)-4a,5,9,10,11,12-Hexahydro-3-methoxy-11-methyl-6H-benzofuro[3a,3,2-ef][2]benzazepin-6-ol, 4-methoxybenzoate (ester)); off-white solid; mp. 183-184°C.

$^1H$ NMR (200 MHz, $CDCl_3$) $\delta$ (ppm) 8.01 (d, *J* = 9.0 Hz, 2H), 8.56 (d, *J* = 8.86 Hz, 2H), 6.69 (d, *J* = 8.18 Hz, 1H), 6.58 (d, *J* = 8.2 Hz, 1H), 6.35 (d, *J* = 10.2 Hz, 1H), 6.0-6.07 (m, 1H), 5.56 (t, *J* = 4.49 Hz, 1H), 4.66 (s, 1H), 4.15 (d, *J* = 15.18 Hz, 1H), 3.89 (s, 3H), 3.84 (s, 3H), 3.68 (d, *J* = 15.18 Hz, 1H), 3.29-3.53 (m, 1H), 3.04-3.12 (m, 1H), 2.73-2.81 (m, 1H), 2.41 (s, 3H), 2.08-2.26 (m, 2H), 1.58-1.66 (m, 1H).

**Example 4:** O-4-Methylbenzoyl-galanthamine (=(4aS,6R,8aS)-4a,5,9,10,11,12-Hexahydro-3-methoxy-11-methyl-6H-benzofuro[3a,3,2-ef][2]benzazepin-6-ol, 4-methylbenzoate (ester)); off-white solid; mp. 71-72°C.

$^1H$ NMR (200 MHz, $CDCl_3$) $\delta$ (ppm) 7.94 (d, *J* = 8.18 Hz, 2H), 7.17 (d, *J* = 8.06 Hz, 2H), 6.69 (d, *J* = 8.18 Hz, 1H), 6.58 (d, *J* = 8.2 Hz, 1H), 6.35 (d, *J* = 9.52 Hz, 1H), 6.0-6.08 (m, 1H), 5.57 (t, *J* = 4.43 Hz, 1H), 4.66 (s, 1H), 4.17 (d, *J* = 15.18 Hz, 1H), 3.89 (s, 3H), 3.70 (d, *J* = 15.18 Hz, 1H), 3.31-3.43 (m, 1H), 3.06-3.13 (m, 1H), 2.74-2.83 (m, 1H), 2.42 (s, 3H), 2.38 (s, 3H), 2.08-2.26 (m, 2H), 1.59-1.67 (m, 1H).

**Example 5:** O-4-Chlorobenzoyl-galanthamine (=(4aS,6R,8aS)-4a,5,9,10,11,12-Hexahydro-3-methoxy-11-methyl-6H-benzofuro[3a,3,2-ef][2]benzazepin-6-ol, 4-chlorobenzoate (ester)); off-white solid; mp.72-74°C.

$^1H$ NMR (200 MHz, $CDCl_3$) $\delta$ (ppm) 7.91 (d, *J* = 8.74 Hz, 2H), 7.27 (d, *J* = 8.72 Hz, 2H), 6.62 (d, *J* = 8.2 Hz, 1H), 6.52 (d, *J* = 8.2 Hz, 1H), 6.30 (d, *J* = 10.34 Hz, 1H), 5.92-6.0 (m, 1H), 5.5 (t, *J* = 4.36 Hz, 1H), 4.59 (s, 1H), 4.09 (d, *J* = 15.18 Hz, 1H), 3.82 (s, 3H), 3.63 (d, *J* = 15.18 Hz, 1H), 3.23-3.46 (m, 1H), 2.99-3.06 (m, 1H), 2.66-2.76 (m, 1H), 2.35 (s, 3H), 2.0-2.2 (m, 2H), 1.52-1.6 (m, 1H).

**Example 6:** O-2-Thenoyl-galanthamine (=(4aS,6R,8aS)-4a,5,9,10,11,12-Hexahydro-3-methoxy-11-methyl-6H-benzofuro[3a,3,2-ef][2]benzazepin-6-ol, thiophene-2-carboxylate (ester)); off-white solid; mp.115-116°C.

$^1H$ NMR (200 MHz, $CDCl_3$) $\delta$ (ppm) 7.78 (dd, *J* = 1.2 Hz, *J* = 3.8 Hz, 1H), 7.51 (dd, *J* = 1.34 Hz, *J* = 4.96 Hz, 1H), 7.04 (dd, *J* = 3.76 Hz, *J* = 4.98 Hz, 1H), 6.69 (d, *J* = 8.18 Hz, 1H), 6.59 (d, *J* = 8.04 Hz, 1H), 6.35 (d, *J* = 10.2 Hz, 1H), 6.02 (dd, *J* = 4.7 Hz, *J* = 10.2 Hz, 1H), 5.54 (t, *J* = 4.49 Hz, 1H), 4.63 (s, 1H), 4.18 (d, *J* = 15.02 Hz, 1H), 3.87 (s, 3H), 3.71 (d, *J* = 15.18 Hz, 1H), 3.31-3.5 (m, 1H), 3.07-3.14 (m, 1H), 2.73-2.83 (m, 1H), 2.42 (s, 3H), 2.04-2.26 (m, 2H), 1.6-1.68 (m, 1H).

**Example 7:** O-5-Chloro-2-thenoyl-galanthamine (=(4aS,6R,8aS)-4a,5,9,10,11,12-Hexahydro-3-methoxy-11-methyl-6H-benzofuro[3a,3,2-ef][2]benzazepin-6-ol, 5-chlorothiophene-2-carboxylate (ester)); off-white solid; mp. 58-59°C.

$^1H$ NMR (200 MHz, $CDCl_3$) $\delta$ (ppm) 7.5 (d, *J* = 4.04 Hz, 1H), 7.80 (d, *J* = 4.02 Hz, 1H), 6.62 (d, *J* = 8.04 Hz, 1H), 6.52 (d, *J* = 8.06 Hz, 1H), 6.31 (d, *J* = 10.2 Hz, 1H), 5.92 (dd, *J* = 4.57 Hz, *J* = 10.2 Hz, 1H), 5.45 (t, *J* = 4.36 Hz, 1H), 4.56 (s, 1H), 4.08 (d, *J* = 15.16 Hz, 1H), 3.81 (s, 3H), 3.61 (d, *J* = 15.18 Hz, 1H), 3.21-3.34 (m, 1H), 2.97-3.04 (m, 1H), 2.64-2.74 (m, 1H), 2.34 (s, 3H), 1.97-2.19 (m, 2H), 1.5-1.57 (m, 1H).

**General procedure 2**

To the solution of the corresponding acid acid (13.87 g, 135.8 mmol) in DCM (250 mL) was added DCC (33.62 g, 162.9 mmol) followed by DMAP (3.32 g, 27.15 mmol), reaction mixture was stirred for additional 30 minutes at room temperature .To this (-)-galanthamine hydrobromide (10.0 g, 27.15 mmol) and triethyl amine (4.6 mL, 32.59 mmol) was added, the mixture was stirred overnight at room temperature under argon. The precipitated DCHU was removed by

filtration and the filtrate was evaporated. The additional DCHU was removed by subsequent trituration with cold ethyl acetate and filtration. The ethyl acetate solution was roto-evaporated and the crude product obtained was purified by column chromatography to give the desired product.

**Example 8:**

2-Methyl-butanoyl-galanthamine (=(4aS,6R,8aS)-4a,5,9,10,11,12-Hexahydro-3-methoxy-11-methyl-6H-benzofuro[3a,3,2-ef][2]benzazepin-6-ol, 2-methyl-butanoate (ester)) was obtained in 53% yield as a solid using the general procedure 2.

The same product, identical in every respect (HPLC, m.p., 1H-NMR), was also obtained in 58% yield using the general procedure 1.

**Example 9:**

2-Methyl-propanoyl-galanthamine (=(4aS,6R,8aS)-4a,5,9,10,11,12-Hexahydro-3-methoxy-11-methyl-6H-benzofuro[3a,3,2-ef][2]benzazepin-6-ol, 2-methyl-propanoate (ester)) was obtained in 63% yield as a solid using the general procedure 1.

**Example 10:**

2-Methyl-propanoyl-galanthamine hydrochloride salt

To the solution of 2-methyl-propanoyl-galanthamine (150 mg, 0.43 mmol) in ethyl acetate (5 mL), ethyl acetate saturated with HCl (5 mL) was added slowly with stirring at 0°C. The reaction mixture was stirred at room temperature for 1 h. Solvent was evaporated and the residue obtained was washed with dry ether and was dried under high vacuum to give 164 mg (97 %) of desired product as an off-white solid.

Anal. calcd for $C_{21}H_{27}NO_4$ (1.5 HCl): C, 61.2; H, 6.97; N, 3.40. Found: C, 61.62; H, 6.95; N, 3.91.

**Example 11:**

2-Methyl-propanoyl-galanthamine citric acid salt

To the solution of 2-methyl-propanoyl-galanthamine (150 mg, 0.43 mmol) in methanol (5 mL), a solution of citric acid in methanol (5 mL) was added slowly with stirring at room temperature. The reaction mixture was stirred at room temperature for 1 h. Solvent was evaporated and the residue obtained was precipitated from methanol-diethyl ether and was dried under high vacuum to give 187 mg (81 %) of desired product as a off-white solid. Anal. calcd for $C_{27}H_{35}NO_{11}$ (1.0 $H_2O$): C, 57.14; H, 6.57; N, 2.47. Found: C, 57.43; H, 6.48; N, 2.53.

**General procedure 3**

To a stirred solution of (-)-galanthamine hydrobromide (1.10 mmol) in pyridine (6 mL) at 0°C under nitrogen, the corresponding acid chloride (2.2 mmol) was added and the mixture was stirred until a TLC showed the reaction to be complete. Then $CH_2CL_2$ (10 mL) and water (10 mL) were added and stirring was continued for 30 min. The organic layer was separated, washed with water (2×10 mL), dried over anhydrous $MgSO_4$ and the solvent removed. The residue was purified by flash chromatography giving the product identical in all respects to the product of example 1.

**Example 12:**

Synthesis of R1-pyridinoyl-galantamine

In addition to the examples provided above, the following compounds were prepared by the described general procedures:

Table 2:

| MF | MW | Substituent R1 |
|---|---|---|
| $C_{21}H_{25}NO_4$ | 355,43 | cyclopropanecarboxylate |
| $C_{24}H_{23}Cl_2NO_4$ | 460,3 | 3,4-dichlorobenzoate |

(continued)

| MF | MW | Substituent R1 |
|---|---|---|
| $C_{28}H_{33}NO_4$ | 447,57 | 4-tert-butylbenzoate |
| $C_{25}H_{23}ClF_3NO_4$ | 493,91 | 4-chloro-3-(trifluoromethyl)benzoate |
| $C_{25}H_{23}F_3N_2O_6$ | 504,46 | 4-nitro-3-(trifluoromethyl)benzoate |
| $C_{25}H_{24}F_3NO_4$ | 459,47 | 4-(trifluoromethyl)benzoate |
| $C_{24}H_{23}Cl_2NO_4$ | 460,36 | 2,4-dichlorobenzoate |
| $C_{24}H_{24}N_2O_6$ | 436,47 | 4-nitrobenzoate |
| $C_{24}H_{24}ClNO_4$ | 425,92 | 3-chlorobenzoate |
| $C_{25}H_{24}F_3NO_4$ | 459,47 | 3-(trifluoromethyl)benzoate |
| $C_{24}H_{24}N_2O_6$ | 436,47 | 3-nitrobenzoate |
| $C_{24}H_{23}Cl_2NO_4$ | 460,36 | 3,5-dichlorobenzoate |
| $C_{26}H_{30}N_2O_4$ | 434,54 | 3-(dimethylamino)benzoate |
| $C_{25}H_{27}NO_4$ | 405,50 | 3-methylbenzoate |
| $C_{24}H_{24}ClNO_4$ | 425,92 | 2-chlorobenzoate |
| $C_{24}H_{23}F_2NO_4$ | 427,45 | 2,4-difluorobenzoate |
| $C_{24}H_{23}Cl_2NO_4$ | 460,36 | 2,5-dichlorobenzoate |
| $C_{24}H_{24}FNO_4$ | 409,46 | 4-fluorobenzoate |
| $C_{26}H_{30}N_2O_4$ | 434,54 | 4-(dimethylamino)benzoate |
| $C_{24}H_{26}N_2O_4$ | 406,49 | 4-aminobenzoate |
| $C_{27}H_{32}N_2O_4$ | 448,57 | 4-(dimethylamino)-3-methylbenzoate |
| $C_{25}H_{25}NO_6$ | 435,48 | 2H-1,3-benzodioxole-5-carboxylate |
| $C_{26}H_{27}N0_5$ | 433,51 | 4-acetylbenzoate |
| $C_{22}H_{23}NO_4S$ | 397,50 | thiophene-3-carboxylate |
| $C_{21}H_{23}N_3O_4$ | 381,44 | 1H-imidazole-5-carboxylate |
| $C_{21}H_{22}N_2O_5$ | 382,42 | 1,3-oxazole-5-carboxylate |
| $C_{21}H_{22}N_2O_4S$ | 398,48 | 1,3-thiazole-5-carboxylate |
| $C_{21}H_{22}N_2O_4S$ | 398,48 | 1,3-thiazole-2-carboxylate |
| $C_{26}H_{27}NO_6$ | 449,51 | 2-(acetyloxy)benzoate |

**Example 13**

Preparation of mouse brain homogenate

The brain is removed from the animal (mouse), shock frozen in liquid nitrogen and stored at minus 80°C until use. Before preparing the extract, the frozen brain is thawed on ice and the weight is determined. Ice cold buffer (130 mM NaCl, 5 mM KCl, 2.5 mM CaCl2, 1 mM MgCl2, 5 mM Glucose, 5 mM HEPES, pH 7.4) is added to the thawed mouse brain (1:4, weight to volume, resulting in a 20 % brain homogenate). The tissue is then homogenized in a potter homogeniser on ice, moving the piston up and down 11 times at 240 rpm. The freshly prepared mouse brain homogenate is divided into aliquots.

**Example 14**

Procedure for measuring the inhibition of brain esterase. Results shown as figure 1

A modified Ellmann's esterase test is used. Briefly the method relies on the cleavage of the substrate acetylthiocholine to acetate and thiocholine. The latter reacts with DTNB (5,5'-Dithiobis-(2-nitro-benzoicacid) to a yellow compound, which can be quantified spectrometrically. The incubation buffer contains 51 mmol/l sodium phosphate buffer and 0.05 % Tween 20, at pH 7.2 and is supplemented with 100 mg/l DTNB, and 0.2 % mouse brain homogenate (prepared as described in Example 13). The compound to be investigated is added to the desired concentration. The mixture is brought to 37°C and the reaction is started by addition of 200 μM acetylthiocholine. $A_{405}$ is measured in 1s intervals in a microplate reader for 40 s. The linear parts of the absorption-time-curves represent the starting speed of the enzymatic reaction and are used for the calculation of the reaction speed. The slope of the curve corresponds to the reaction speed. The inhibition is expressed as per cent of the non inhibited reaction according to following equation:

$$\% \text{ Inhibition} = 100 * (1-(\text{Slope}_{\text{inhibited}} / \text{Slope}_{\text{noninhibited}})).$$

**Example 15**

Procedure for determination of prodrug cleavage in mouse brain homogenate.

To aliquots of mouse brain homogenate as prepared according to Example 13 pro-galantamine derivatives are added and adjusted to a final concentration of 10 μM of the prodrug. At the end of the incubation time, 12 μl 0.1 M NaOH and 100 μl saturated KCl are added to the 0.1 ml reaction mixture and mixed thoroughly. The remaining prodrug and the released galantamine are extracted by 200 μl toluene. The toluene extraction step is repeated twice using 150 μl toluene and the obtained extracts are pooled, dried, dissolved in 50 % Methanol and used for subsequent HPLC analysis.

**Example 16**

Investigation of the allosteric modulation effect of drug candidates on nicotinic acetylcholine receptors (nAChRs) expressed in HEK-293 cells by electrophysiology. Results shown as figure 3

Single HEK-293 cells expressing either human α4β2, human α3β4, or chimeric chicken α7 (with mouse $5HT_3$) nAChR were plated on fibronectin-coated cover slips for 3 days before measurement. The selected nAChR containing cells were placed in the recording bath, filled with extracellular buffer (145 mM NaCl, 5 mM KCl, 1 mM $MgCl_2$, 2 mM $CaCl_2$, 10 mM D-glucose, 10 mM HEPES, pH 7.3, approximately 300 mOsm). Patch-clamp system consisted of an inverted microscope (Zeiss, Germany), computer-controlled patch-clamp amplifier with PatchMaster software (HEKA, Germany), tubing perfusion system (ALA, USA) together with a U-tube applicator (IMM, Germany) and dual micromanipulators. The patch pipettes were pulled from fire-polished, 100 mm long and 1.5 mm width, single borosilicate glass capillaries (WPI, Germany). A programmable puller (Sutter, USA) was used to prepare a twin pair of ready for use pipettes. Each patch pipette (resistance 4-8 MΩ) was used only once. Pipettes were filled with an internal buffer (140 mM CsCl, 11 mM EGTA, 10 mM HEPES, 2 mM $MgCl_2$, pH 7.3, approximately 300 mOsm) and connected to the working electrode. Working and reference electrodes for experiments were made from daily renewed, freshly chlorinated silver wire (40 mm x 0.4 mm) and were connected to a headstage circuit of the patch-clamp amplifier. Patching was done using rectangular test pulses with an amplitude of -1 mV and a duration of 20 ms. Immediately after formation of the gigaseal the holding potential of -70 mV was applied to the patch electrode and whole-cell recordings were established by using negative pressure pulses. All necessary compensations for fast and slow membrane capacitance and serial resistance transients were automatically set within the PatchMaster software. Whole-cell currents were evoked by the application of nicotine at the $EC_{50}$ for each appropriate nAChR subtype (α4β2 and α3β4 $EC_{50}$ = 30 μM, chimeric α7 $EC_{50}$ = 3 μM). To evaluate an allosteric potentiating ligand (APL) effect of selected compounds on each subtype of nAChR, they were added to stimulating nicotine solutions at the following concentrations: 1, 5, 10, 50, 100, 500, 1,000, 5,000 and 10,000 nM, and solutions were applied to the cell surface during 500 ms pulses through the U-tube, and then corresponding currents, digitized to 10 kHz, were recorded for 10 s. Consecutive current stimulations were done with a 2 min interval to avoid nAChR desensibilisation and to ensure full exchange of stimulating solutions. The averaged peak amplitudes of the currents, measured in the presence of selected compound concentrations, were compared with those determined in the absence of compounds (control) and they were calculated as % of control. The measurements of an APL effect of particular compound were repeated on a minimum of five cells to obtain the mean values +/- SD. Mean values of the observed APL effect, which did not exceed 15% were treated as insignificant. To present a concentration-dependent APL effect of particular compound, the corresponding % of control values +/- SD were plotted against the concentrations used.

**Example 17**

Pharmacokinetics of pro-galantamine Gln-1062 (3mg/kg) in the mouse, results shown as figure 4

STUDY OBJECTIVE

Determination of the pharmacokinetic profiles of Gln-1062 and its cleavage product galantamine in blood and brain. Determine the brain-to-blood concentration ratios of Gln-1062 and its cleavage product galantamine, and assess the blood-brain barrier penetration capacities.

STUDY PLAN

Bioanalysis

Analytical method for estimation of Gln-1062 was evaluated for its linearity, precision & accuracy and recovery in SAM blood and brain homogenate using LC/MS/MS.

LC/MS/MS Parameters

The parameters of chromatographic conditions and extraction conditions for the Gln-1062 and Galantamine analysis were

Chromatographic parameters:

| | |
|---|---|
| Column | : Phenomenex Synergi, Polar-RP 80 A, C18, 75 x 2.0 mm, 4μ |
| Mobile Phase | |
| Mobile Phase Buffer | : 40 mM Ammonium Formate, pH 3.5 |
| Aqueous Reservoir (A) | : 10% Buffer, 90% Water |
| Organic Reservoir (B) | : 10% Buffer, 90% Acetonitrile |
| Flow rate | : 0.450 mL/min |

Gradient Programme :

| Time (min) | Gradient Curve | %A | %B |
|---|---|---|---|
| 0 | 1 | 100 | 0 |
| 1.2 | 1 | 60 | 40 |
| 3 | 1 | 0 | 100 |
| 3.1 | 1 | 100 | 0 |
| 5 | 1 | 100 | 0 |

Divert valve time shedule:

| Time (min) | Divert Valve | |
|---|---|---|
| | Waste | MS |
| 0 | x | |
| 1.2 | | x |
| 4.5 | x | |

| | | |
|---|---|---|
| Run time | : 5.0 min | |
| Column oven temperature | : Ambient | |
| Auto sampler temperature | : Ambient | |
| Auto sampler Wash | :Water:acetonitrile:isopropanol with 0.2% formic acid,1:1:1(v/v/v) | |
| Retention time | : Gln-1062 | : 3.33 ± 0.05 min. |

(continued)

| | |
|---|---|
| Galantamine | : 2.44 ± 0.05 min |
| Metoprolol | : 2.80 ± 0.05 min. |

Mass Parameters (API 3200):

| | |
|---|---|
| Mode | : MRM |
| Polarity | : Positive |
| Ion source | : Turbo spray |
| Analyte | : Gln-1062 (Q1 Mass 392.4 ; Q3 Mass 213.2) |
| | Galantamine (Q1 Mass 288.3 ; Q3 Mass 213.1) |
| ISTD | : Metoprolol (Q1 Mass 268.4 ; Q3 Mass 116.2) |

Source/ Gas Parameters:

| | |
|---|---|
| Curtain gas (CUR) | 10 |
| Collision gas | |
| (Collision associated Dissociation) CAD | 5 |
| Ion Spray Voltage (IS) | 5500 V |
| Temperature (TEM) | 575 °C |
| GS1 | 55 |
| GS2 | 45 |
| Ihe | ON |

Compound Parameters:

| Parameter | Galantamine | Metoprolol | Gln-1062 |
|---|---|---|---|
| Declustering Potential (V) | 45 | 35 | 50 |
| Entrance Potential (V) | 10 | 10 | 10 |
| Collision Cell Entrance Potential (V) | 20 | 20 | 20.00 |
| Collision Energy (eV) | 32 | 26 | 32 |
| Collision Cell Exit Potential (V) | 4.5 | 2 | 5 |
| Dwell Time (milliSec) | 200 | 200 | 200 |

Extraction Procedure:

A Preparation of STD, QC and Study samples

To 50μL of blood/brain homogenate study/spiked sample 150μL of cold acetonitrile containing Metoprolol (100.03ng/mL) was added

↓

Vortexed for 30 seconds.

↓

(continued)

Centrifuged at 13000 rpm for 10 min at 4 °C

↓

100 μL of supernatant diluted with 100μL of milli-Q water

↓

Vortexed and transferred into pre-labeled auto sampler vials.

B Preparation of calibration curve standards for recovery

To 50μL of balnk blood/brain homogenate 150μL of cold acetonitrile containing Metoprolol (100.03ng/mL) was added

↓

Vortexed for 30 seconds.

↓

Centrifuged at 13000 rpm for 10 min at 4 °C

↓

Separate the supernant

↓

Add 2μL of Spiking solution-II to 198 μL of above supernant and vortex

↓

100 μL of above mixture diluted with 100μL of milli-Q water

↓

Vortexed and transferred into pre-labeled auto sampler vials

Method evaluation

This method was evaluated for linearity, precision & accuracy and recovery of Gln-1062 in SAM blood and brain homogenate.

A. Linearity, precision & accuracy

A single standard curve and six replicates each of three quality control (QC) levels (18 total QCs) were extracted and analyzed. The linearity of the calibration curve was determined by a weighed least square regression analysis.

Acceptance Criteria

i. At least six out of nine standards must have an accuracy of ±15% from nominal, except at the lower limit of quantitation (LLOQ) where ±20% is acceptable.

ii. Two-thirds of the batch QCs and at least half of the QCs at each level must have a calculated accuracy of ±15% from nominal.

iii. Intra-assay Mean Precision and Accuracy

   1. Four out of six QCs must be available to determine accuracy and precision.

   2. The intra-assay coefficient of variation (%CV) of each QC level must not exceed 15% and the accuracy of the mean value for each validation to be accepted.

Gln-1062 linearity, precision and accuracy in blood and brain homogenate matrices shown in Table 1 & 2, respectively.

B. Recovery

The recovery of Gln-1062 from the blood and brain homogenate matrices were also evaluated.

Recovery was determined by quantifying the concentration of the analytes in extracted matrix QC samples with a standard curve prepared in post-extract (blank extracts) sample matrix as described in 5.3 B

Gln-1062 recovery in blood and brain homogenate matrices shown in Table 3 & 4, respectively.

Animal study

Study design

| Animal | Test item | Dose (mg/kg) | Dose Conc. (mg/mL) | Dose Volume (mL/kg) | Dose route | No. of Animals for each time point | Sample time points (hr) |
|---|---|---|---|---|---|---|---|
| Male SAM 25-33 gm | Gln-1062 | 3 | 0.2 | 15 | i.v, bolus (tail vein) | 3 | Predose, 0.05, 0.10, 0.17,0.33, 0.50,0.83,1.33, 2.0 and 4.0 |

Sample collection

Collection of Blood:

Blood samples were collected from the retro-orbital plexus. 0.5 ml of blood was collected into a pre-labeled polypropylene micro centrifuge tube containing sodium citrate as the anticoagulant, and kept on ice.

Blood was mixed gently with anticoagulant and an aliquot of 50μL of blood sample immediately precipitated as described in the section 5.3A. Remaining volume of blood sample at each time point frozen on dry ice.

All the blood samples were transferred to analytical department and frozen at - 80 ± 10 °C until analysis.

Collection of Brain:

Immediately after blood withdrawal, brain was perfused with phosphate buffer saline (pH 7.4), removed and frozen on dry ice.

All the brain samples were transferred to analytical department and frozen at - 80 °C until analysis.

Brain homogenate Preparation:

Brain samples were thawed on ice and weighed. An appropriate volume ice cold homogenizing media (methanol: water::20:80,v/v) added. On ice, homogenized the brain sample with the polytron homogenizer and make up the volume with homogenizing media to get 1 gm of brain per 4 mL of homogenate. After homogenizing, immediately freezed the brain homogenate samples at -80°C until analysis.

**Example 18**

Behavioural index for gastro-intestinal side effects in ferrets following application of galantamine and several R1-pro-galantamines, respectively. Results shown in figure 5.

Test system

Fourteen adult male *Putoris furo* ferrets (Marshall BioResources (North Rose, USA)), weighting 750-1000 grams on the day of experimentation were used in the present study. In agreement with the sponsor four out of the fourteen animals were included in two experimental groups (Tables 3 and 4, ferrets # 1, 2, 3 and 4).

Animal housing

The acclimatization of the animals lasted at least 5 days. At receipt, animals were collectively housed in cages at Syncrosome's premises. They had free access to food and drinking water *ad libitum.*

Test item and Reference compound

During this study, one reference compound (Galantamine) and one Galantos candidate compound (GLN979) were tested at two doses each. Both compounds were administered I.P. at doses and concentrations which are detailed in table 1.

Details of the different compound shipments are presented in table 2.

| Receipt / Compoun | November, the 11th,2007 | November, the 21th, 2007 | November, the 29th, 2007 |
|---|---|---|---|
| Galantamine | 48,0 mg | 256.0 mg (One vial) | - |
| GLN979 | 48,0 mg | - | 262.6 mg |
| 2-Hydroxypropyl-β-cyclodextrin | U.I : (One vial) | 7.3 g + 7.3 g (Two vials) | ≈ 4.7g + ≈ 5.0 g |
| NaCl | U.I : (One vial) | 3.0 g (One vial) | - |

As requested by the sponsor, the same vehicle (15% 2-Hydroxypropyl-β-cyclodextrin / 96 mM NaCl) was used for both Galantamine and GLN979 preparation. A detailed solubilization protocol was sent by mail by Galantos to Syncrosome and received on November 5th, 2007.

Test compound (GLN979)

| | |
|---|---|
| Nature | GLN979. |
| Molar mass | U.I. |
| Administration dose | 20 and 40 mg/kg B.W. |
| Administration route | I.P. |
| Vehicle | 15% 2-Hydroxypropyl-β-cyclodextrin / 96 mM NaCl. |

Reference compound (Galantamine)

| | |
|---|---|
| Nature | Galantamine |
| Molar mass | U.I. |
| Administration dose | 3 and 20 mg/kg B.W. |
| Administration route | I.P. |
| Vehicle | 15% 2-Hydroxypropyl-β-cyclodextrin / 96 mM NaCl. |

I.P. administrations

For the 4 experimental groups, the administrations were performed in unanaesthetized animals through the I.P. route at To (figure 1). The volumes of administration for each group are detailed in table 1.

Emesis test

After I.P. administration of the compound solution, the animals were continuously observed by a trained technician for four hours. During that period, the number of episodes of vomiting (series of retches leading to the expulsion of part of the gastro-intestinal content) were recorded.

Behavioural observation

During the 4 hours-observation period, several side-effects (salivation, shivering, respiratory problems and diarrhea) were also observed. For each of these behaviours, a scoring method was determined with the sponsor. Depending on its severity, each parameter were quantified as:

- *"None"* (None) : Behaviour not observed.
- *"Moderate"* (Mod.) : Behaviour observed with a low frequency and/or a low intensity.
- *"Intense"* (Int.) : Behaviour observed frequently and/or continuously and/or at a high intensity.

Inclusion criteria

All the animals receiving I.P. administration of the reference or the test compound were included in the study regardless of the pattern of both their emetic responses and behavious.

**Example 19**

Reversal from scopolamine-induced amnesia in mice, results shown as figure 6

Drug preparation

Gln 1062, Gln 0979 and galanthamine were dissolved in 15% -hydroxypropyl-β-cyclodextrin in 96 mM NaCl (isotonic) supplied by the sponsor. Gln 1062 and Gln 0979 were used in concentrations of 0.0.1, 0.03, 0.1 and 0.2 mg/ml, which when given in a volume of 10 ml/kg result in doses of 0.1, 0.3, 1 and 2 mg/kg i.p., respectively. Galanthamine was used in concentrations of 0.03, 0.1, 0.2 and 0.5 mg/ml, which when given in a volume of 10 ml/kg result in doses of 0.3, 1, 2 and 5 mg/kg i.p., respectively. Control animals received 15% -hydroxypropyl-β-cyclodextrin in 96 mM NaCl injection as a vehicle.

Nicotine ((-)-Nicotine hydrogen tartrate salt, Sigma, France), scopolamine (-(-)scopolamine hydrochloride, Sigma, France) were dissolved in saline (0.9% NaCl, Aguettant, France) at the concentration of 0.04 and 0.1 mg/ml, respectively. They were administrated at a dosage volume of 10 ml/kg to achieve doses of 0.4 and 1 mg/kg, respectively.

Test animals

Four to five week old male CD-1 mice (Janvier; Le Genest St Isle - France) were used for the study.

They were group-housed (10 mice per cage) and maintained in a room with controlled temperature (21-22°C) and a reversed light-dark cycle (12h/12h; lights on: 17:30 - 05:30; lights off: 05:30 - 17:30) with food and water available ad libitum.

Experimental design

The potential cognitive enhancing property of Gln 1062, Gln 0979 and galanthamine were evaluated in scopolamine-treated mice in the T-maze alternation model under the same experimental conditions. Both Gln 1062 and Gln 0979 were tested in doses of 0.1, 0.3, 1 and 2 mg/kg i.p. Galanthamine was tested in doses 0.1, 0.3, 1, 2 and 5 mg/kg i.p. Nicotine was tested in a dose of 0.4 mg/kg i.p. All these compounds were administrated immediately after the injection of 1 mg/kg i.p. scopolamine (20 min prior to the T-maze trial) used to induce memory deficit.

Memory performance was assessed by the percentage of spontaneous alternation in the T-maze. The number of alternation in saline-injected mice was used as the base level of unaltered memory performance.

Mice were housed 10 per cage. Each mouse in a cage was randomly assigned by a unique number (1 to 10) written on the tail with permanent ink.

Gln 1062, Gln 0979 and galanthamine were tested separately in three set of experiments with different animals. Each set of experiments was divided in series of daily experiments that always comprises at least one representative of each of Saline/vehicle, Scopolamine/vehicle and Scopolamine/Nicotine (0.4mg/kg) groups.

Measurement

The T-maze apparatus was made of gray Plexiglas with a main stem (55 cm long × 10 cm wide × 20 cm high) and two arms (30 cm long × 10 cm wide × 20 cm high) positioned at 90 degree angle relative to the left and right of the main stem. A start box (15 cm long × 10 cm wide) was separated from the main stem by a guillotine door. Horizontal

doors were present to close off specific arms during the force choice alternation task.

The experimental protocol consists of one single session, which starts with 1 "forced-choice" trial, followed by 14 "free-choice" trials. In the first "forced-choice" trial, the animal is confined 5 s in the start arm and then released while either the left or right goal arm is blocked by a horizontal door. After the mouse is released, it will negotiate the maze and eventually enter the open goal arm, and return to the start position. Immediately after the return of the animal to the start position, the closed goal door is opened and the animal is now free to choose between the left and right goal arm ("free choice trials"). The animal is considered as entered when it places its four paws in the arm. A session is terminated and the animal is removed from the maze as soon as 14 free-choice trials have been performed or 10 min have elapsed, whatever event occurs first. The average duration of a trial is 6 min.

The apparatus is cleaned between each animal using alcohol (70°). Urine and feces are removed from the maze.

During the trials, animal handling and the visibility of the operator are minimized as much as possible.

The percentage of alternation over the 14 free-choice trials was determined for each mouse and was used as an index of working memory performance. This percentage was defined as entry in a different arm of the T-maze over successive trials (i.e., left-right-left-right, etc).

Statistical analysis

Analysis of variance (ANOVA) was performed on the result data. Fisher's Protected Least Significant Difference was used for pairwise comparisons. p value $\leq 0.05$ were considered significant. The drug induced reversion of scopolamine-induced memory deficit was calculated by setting the respective response of the saline/vehicle group as 100% and the scopolamine/vehicle group as 0% reversion.

In order to determine the $ED_{50}$ for each drug, the recovery performance was plotted following a sigmoidal dose-response model (graphpad software). ED50 was read from the curve fitting table and represents the effective dose associated with 50% of response.

**Table A**

| molregno molstructure | abbrev. |
|---|---|
| 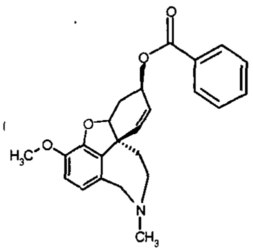 | Bz-Gal |
| 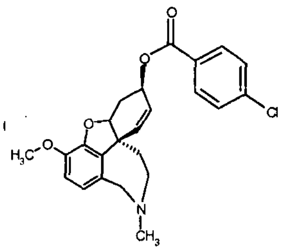 | 4-Cl-Bz-Gal |
| 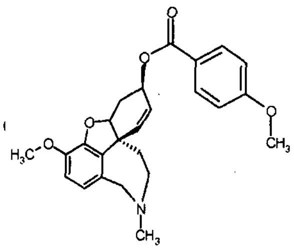 | 4-MeO-Bz-Gal |

(continued)

| molregno molstructure | abbrev. |
|---|---|
| 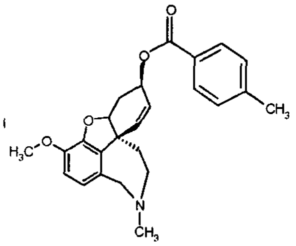 | 4-Me-Bz-Gal |
| 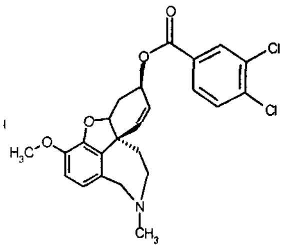 | 3,4-Cl2-Bz-Gal |
| 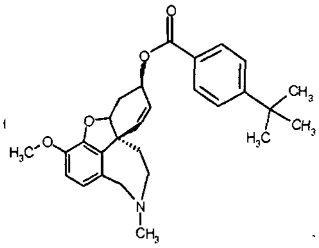 | 4-tBu-Bz-Gal |
| 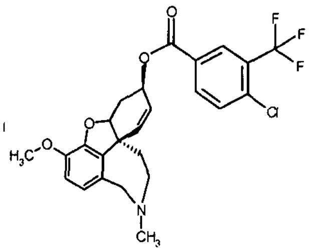 | 3-CF3-4-Cl-Bz-Gal |
| 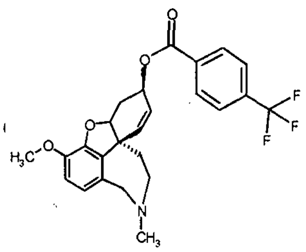 | 4-CF3-Bz-Gal |
| 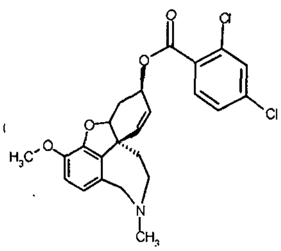 | 2,4-Cl2-Bz-Gal |

(continued)

| molregno | molstructure | abbrev. |
|---|---|---|
| | 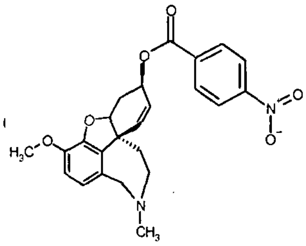 | 4-NO2-Bz-Gal |
| | 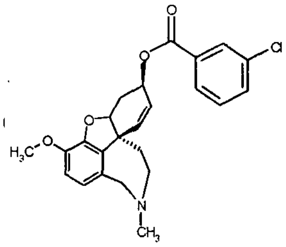 | 3-Cl-Bz-Gal |
| | 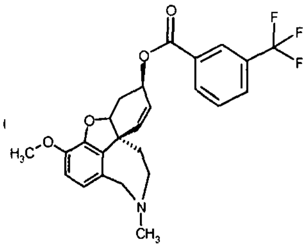 | 3-CF3-Bz-Gal |
| | 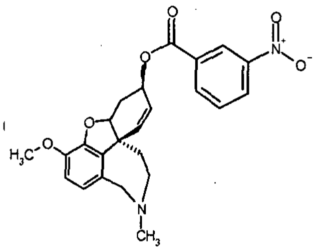 | 3-NO2-Bz-Gal |
| | 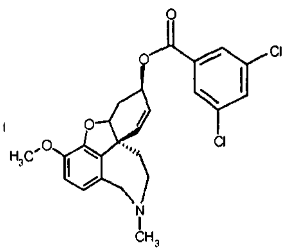 | 3,5-Cl2-Bz-Gal |
| | 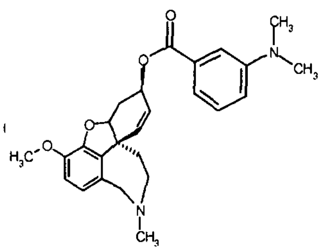 | 3-Me2N-Bz-Gal |

(continued)

| molregno molstructure | abbrev. |
|---|---|
| 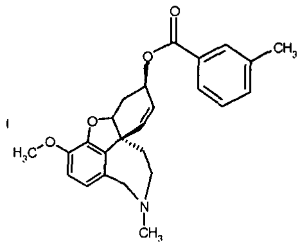 | 3-Me-Bz-Gal |
| 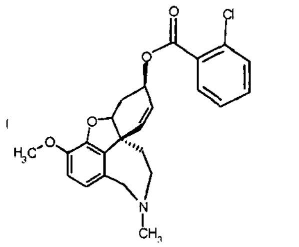 | 2-Cl-Bz-Gal |
| 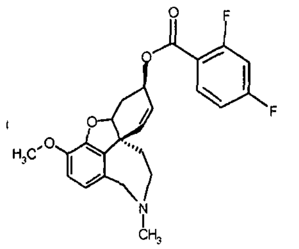 | 2,4-F2-Bz-Gal |
| 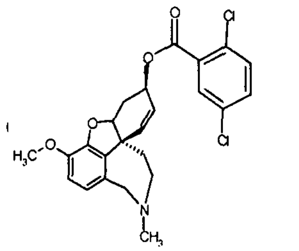 | 2,5-Cl2-Bz-Gal |
| 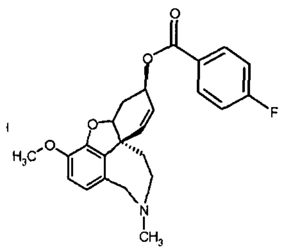 | 4-F-Bz-Gal |
| 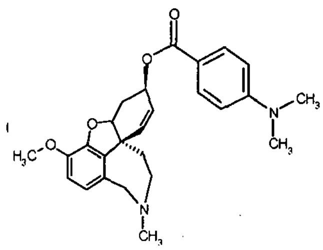 | 4-NMe2-Bz-Gal |

(continued)

| molregno molstructure | abbrev. |
|---|---|
| 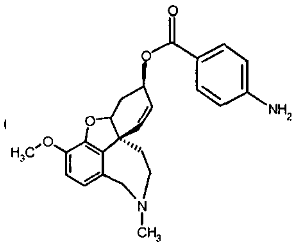 | 4-NH2-Bz-Gal |
| 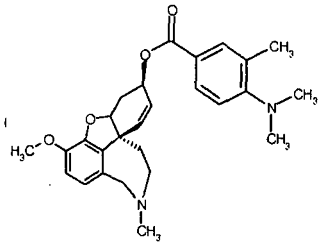 | 3-Me-4-NMe2-Bz-Gal |
| 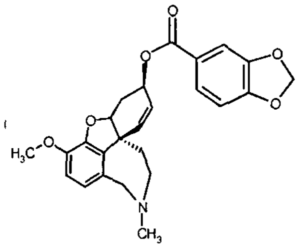 | 3,4-OCH2O-Bz-Gal |
| 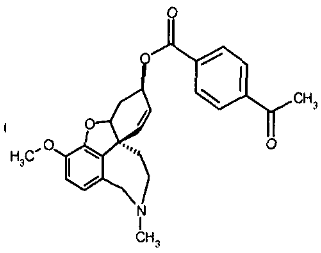 | 4-Ac-Bz-Gal |
| 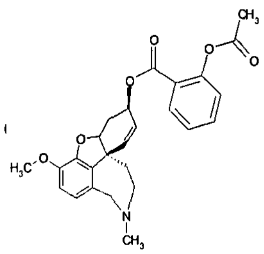 | 2-AcO-Bz-Gal |
| 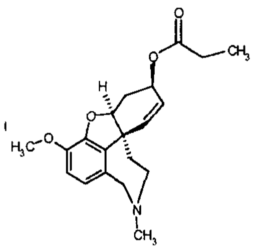 | n-prop-Gal |

(continued)

| molregno molstructure | abbrev. |
|---|---|
| 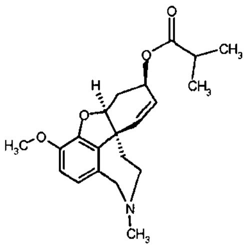 | i-but-Gal |
| 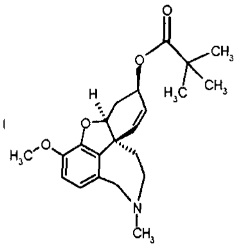 | |
| 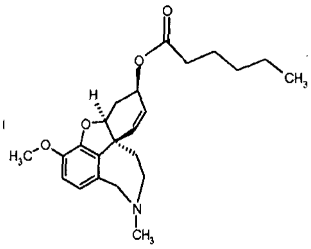 | n-Hex-Gal |
| 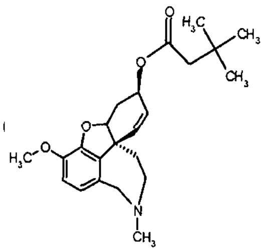 | neo-pent-Gal |
| 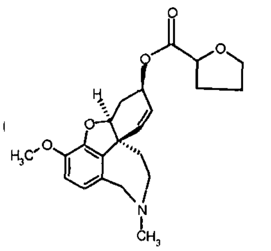 | |
| 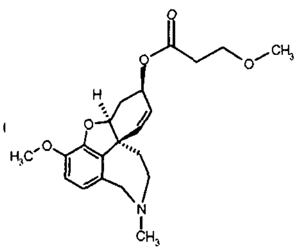 | |

(continued)

| molregno molstructure | abbrev. |
|---|---|
| 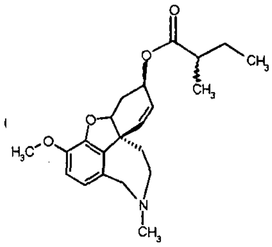 | R/S-i-pent-Gal |
| 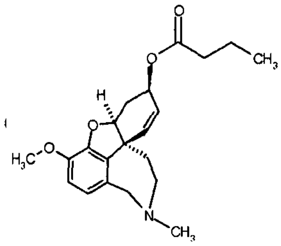 | |
| 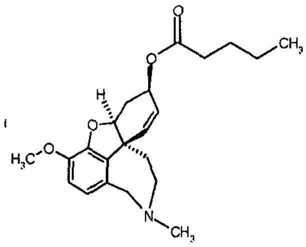 | |
| 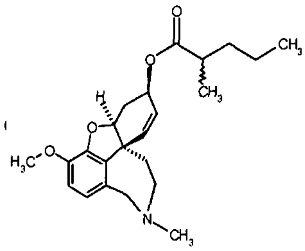 | |
| 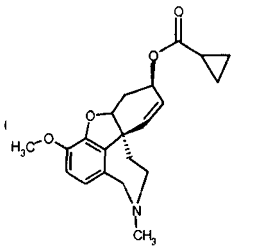 | CyBu-Gal |
| 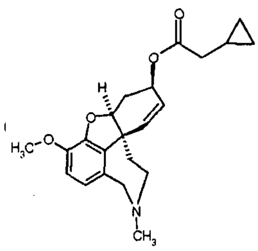 | |

(continued)

| molregno molstructure | abbrev. |
|---|---|
| 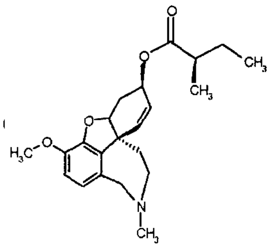 | R/S-i-pent-Gal |
| 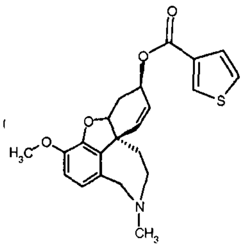 | 3-Th-Bz-Gal |
| 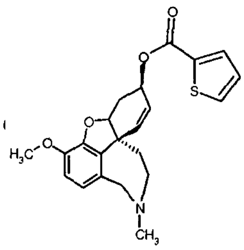 | 2-Th-Bz-Gal |
| 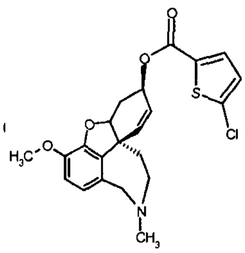 | 5-Cl-2-Th-Bz-Gal |
| 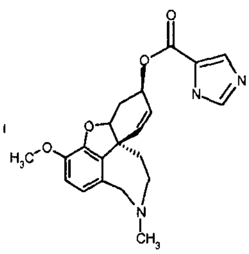 | 5-Im-Bz-Gal |
| 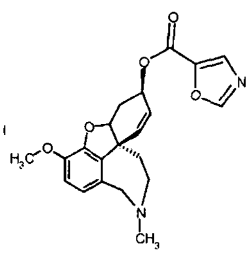 | 5-OA-Bz-Gal |

(continued)

| molregno molstructure | abbrev. |
|---|---|
| 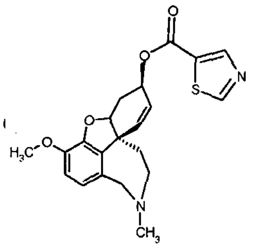 | 5-Th-Bz-Gal |
| 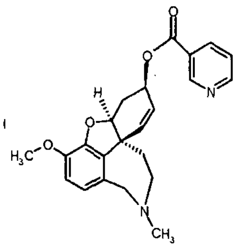 | Nic-Gal |
| 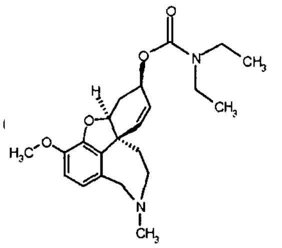 | |

## Claims

**1.** Galantamine pro-drug according to the following formula

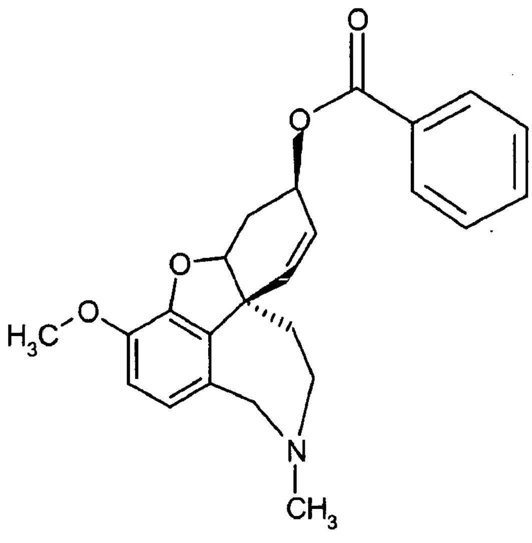

[GLN 1062]

for use as a medicament in the treatment of a neurodegenerative or psychiatric or neurological disease associated with a cholinergic deficit, wherein the disease is selected from Alzheimer's and Parkinson's disease, other types of dementia, schizophrenia, epilepsy, oxygen and nutrient deficiencies in the brain after hypoxia, anoxia, asphyxia, cardiac arrest, various types of poisoning, anesthesia, particularly neuroleptic anesthesia, autism, postoperative delirium and/or subsyndronal postoperative delirium, subsequences of the abuse of alcohol and drugs, addictive alcohol and nicotine craving, and subsequences of radiotherapy.

2. Pharmaceutical composition comprising a galantamine pro-drug according to GLN 1062 for use as a medicament in the treatment of a neurodegenerative or psychiatric or neurological disease associated with a cholinergic deficit according to claim 1.

**Patentansprüche**

1. Galantamin-Prodrug gemäß folgender Formel

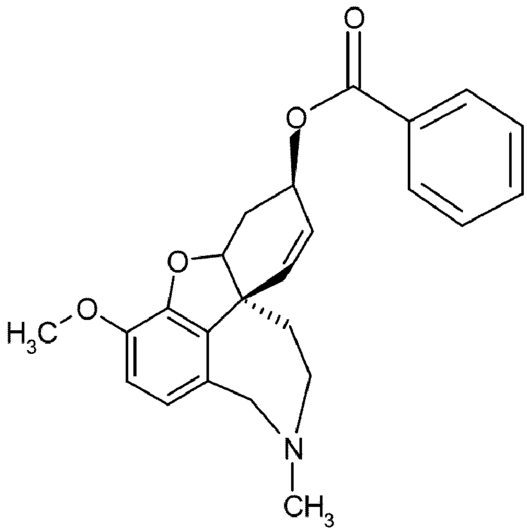

[GLN 1062]

zur Verwendung als Arzneimittel in der Behandlung einer neurodegenerativen oder psychiatrischen oder neurologischen Krankheit, die mit einem cholinergischen Defizit assoziiert ist, wobei die Krankheit aus der Gruppe ausgewählt wird umfassend Alzheimer- und Parkinson-Krankheit, andere Arten von Demenz, Schizophrenie, Epilepsie, Sauerstoff- und Nährstoffmangel des Gehirns nach einer Hypoxie, Anoxie, Asphyxie, Herzstillstand, verschiedene Arten von Vergiftungen, Anästhesie, insbesondere neuroleptische Anästhesie, Autismus, postoperatives Delirium und/oder subsyndronales postoperatives Delirium, Folgen von Alkohol- und Drogenmissbrauch, Alkohol- und Nikotinsucht, und folgen von Radiotherapie.

2. Pharmazeutische Zusammensetzung umfassend eine Galantamin-Prodrug gemäß GLN 1062 zur Verwendung als Arzneimittel in der Behandlung einer neurodegenerativen oder psychiatrischen oder neurologischen Krankheit, die mit einem cholinergischen Defizit assoziiert ist, gemäß Anspruch 1.

**Revendications**

1. Précurseur de médicament à base de galantamine répondant à la formule suivante :

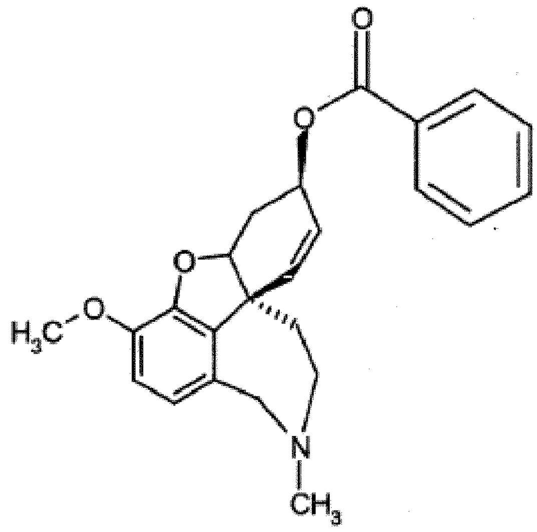

[GLN 1062]

pour son utilisation comme médicament dans le traitement de maladies neurodégénératives ou psychiatriques ou neurologiques associées à un déficit cholinergique, la maladie étant choisie parmi la maladie d'Alzheimer et la maladie de Parkinson, d'autres types de démences, la schizophrénie, l'épilepsie, le manque d'oxygène et de nutriments dans le cerveau après une hypoxie, une anoxie, une asphyxie, un arrêt cardiaque, divers types d'empoisonnements, une anesthésie, en particulier une anesthésie neuroleptique, l'autisme, un délire postopératoire et/ou un délire postopératoire subsyndromal, des conséquences de l'abus d'alcool et de médicaments, un état de manque toxicomanogène d'alcool et de nicotine, et des suites d'une radiothérapie.

**2.** Composition pharmaceutique comprenant un précurseur de médicament à base de galantamine selon GLN 1062, pour son utilisation comme médicament dans le traitement d'une maladie neurodégénérative ou psychiatrique ou neurologique associée à un déficit cholinergique selon la revendication 1.

Figure 1

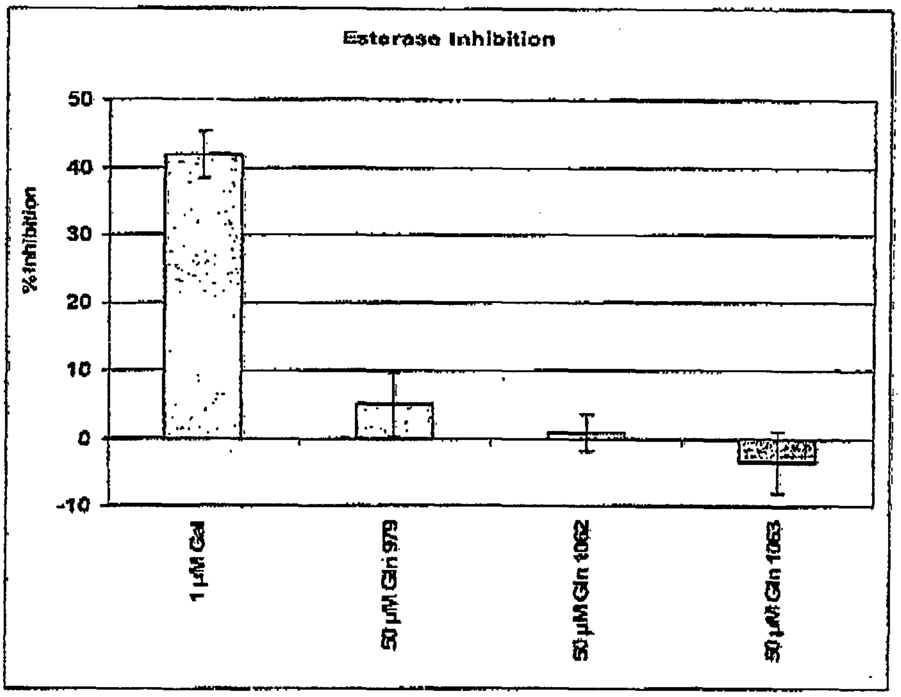


Figure 2

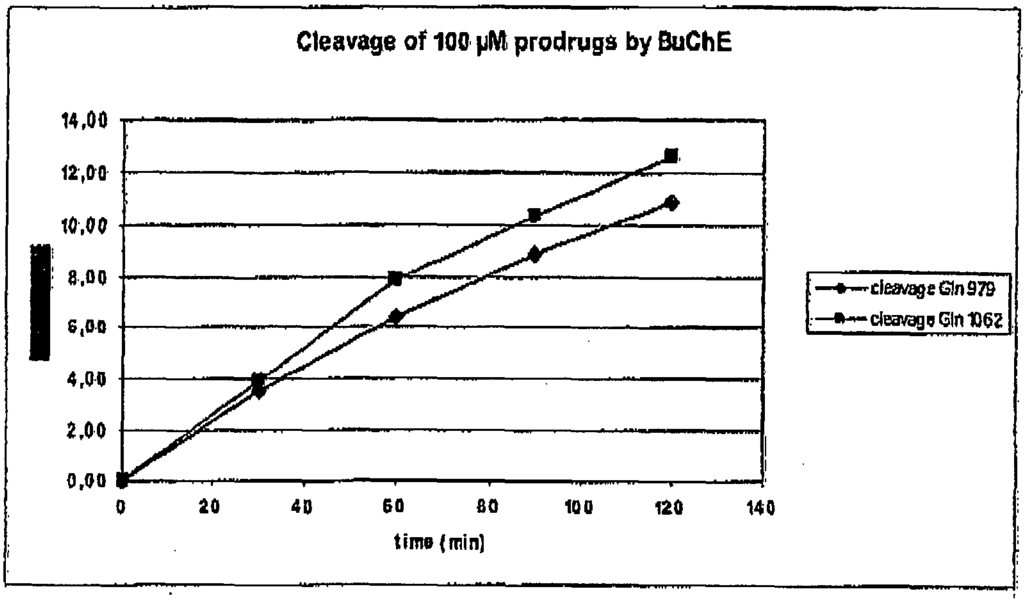

Figure 3

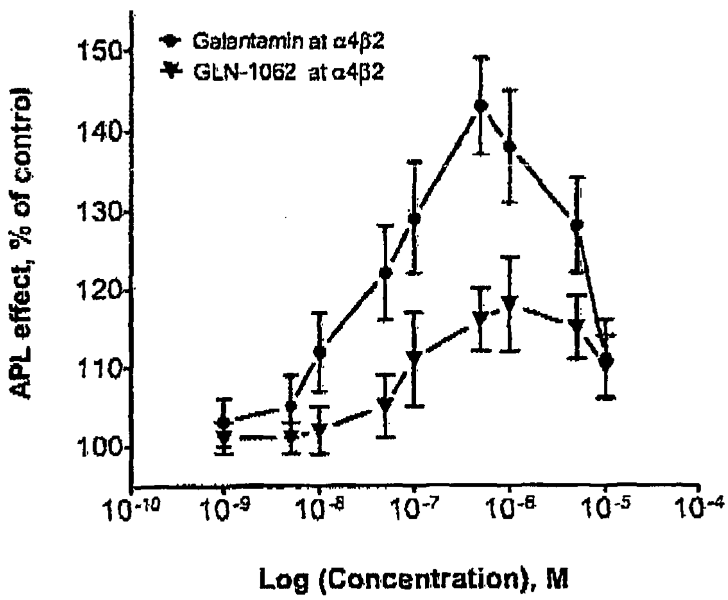
Galantamin at α4β2
GLN-1062 at α4β2
APL effect, % of control
150
140
130
120
110
100
10-10
10-9
10-8
10-7
10-6
10-5
10-4
Log (Concentration), M

Figure 4a

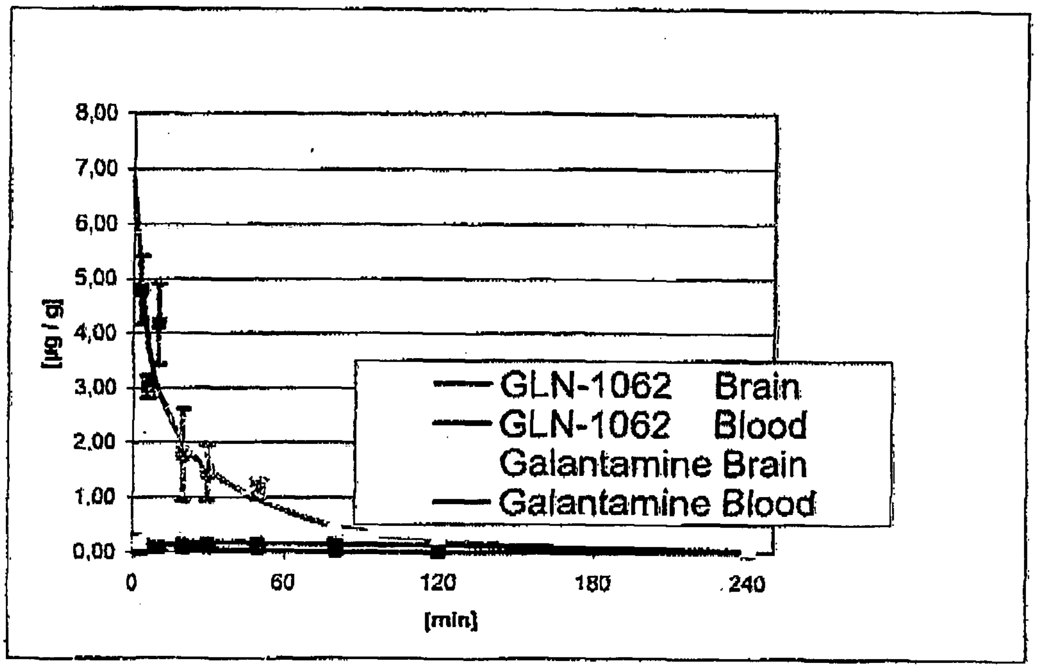


Figure 4b

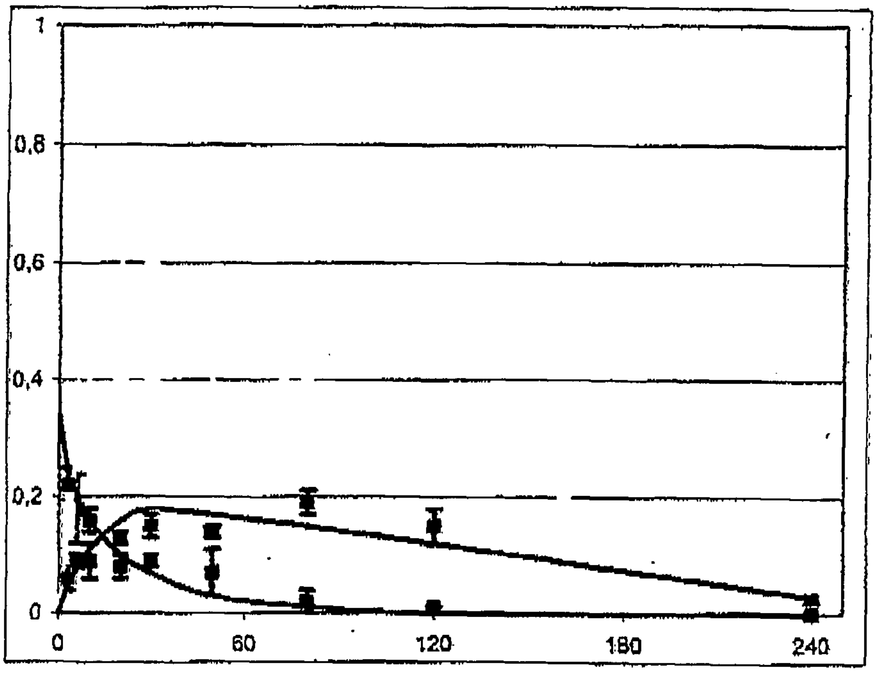

Figure 5

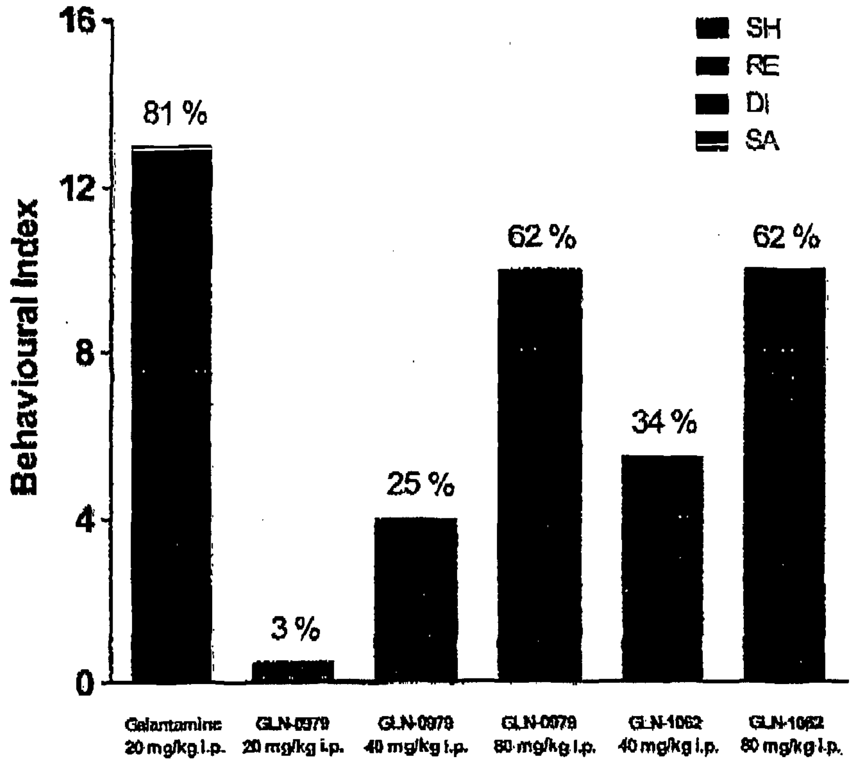
SH
RE
DI
SA
16
12
8
4
0
Behavioural Index
81 %
3 %
25 %
62 %
34 %
62 %
Galantamine 20 mg/kg i.p.
GLN-0979 20 mg/kg i.p.
GLN-0979 40 mg/kg i.p.
GLN-0979 80 mg/kg i.p.
GLN-1062 40 mg/kg i.p.
GLN-1062 80 mg/kg i.p.

Figure 6

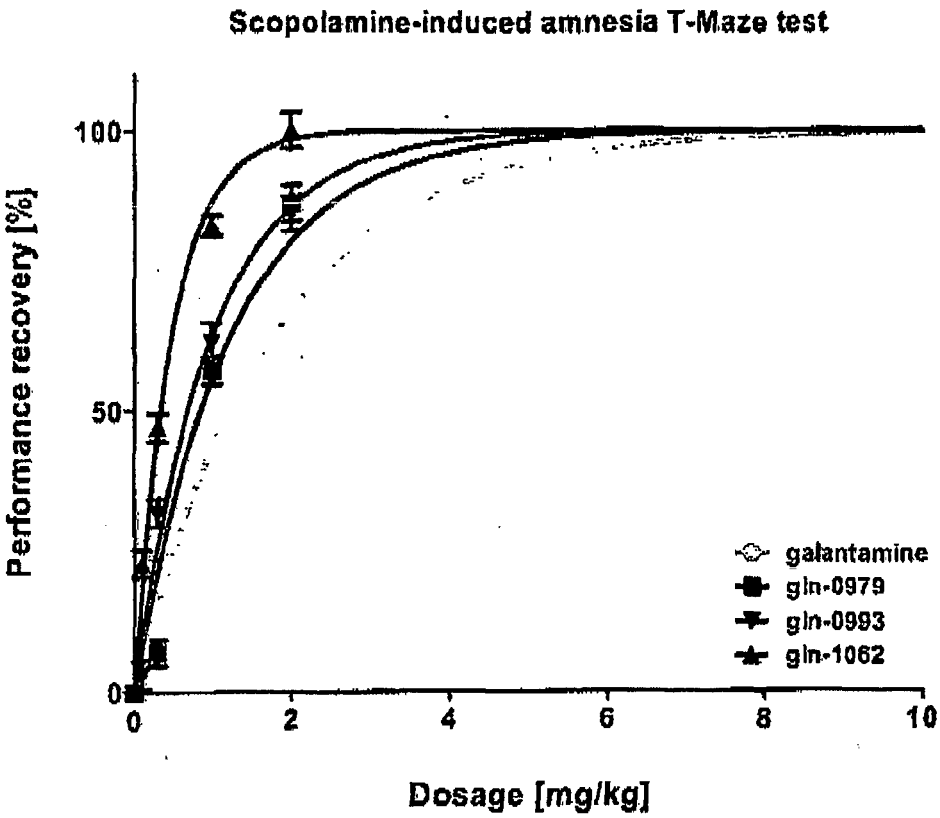


| | Galantamine | GLN-0979 | GLN-0993 | GLN-1062 |
|---|---|---|---|---|
| $EC_{50}$ | 1.2 mg/kg | 0.9 mg/kg | 0.7 mg/kg | 0.3 mg/kg |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007039138 A
- EP 1777222 A
- EP 648771 A
- EP 649846 A
- EP 653427 A
- US 6150354 A
- WO 0174820 A
- WO 0032199 A
- WO 2005030333 A
- WO 8808708 A
- WO 9921561 A
- WO 0143697 A
- US 20030162770 A
- WO 2005030713 A
- WO 9740049 A
- WO 9921561 A1


### Non-patent literature cited in the description


- **HAN, SO YEOP ; MAYER, SCOTT C. ; SCHWEIGER, EDWIN J. ; DAVIS, BONNIE M. ; JOULLIE, MADELEINE M.** Synthesis and biological activity of galanthamine derivatives as acetylcholinesterase (AChE) inhibitors. *Dep. Chem.*
- *Bioorganic & Medicinal Chemistry Letters,* 1991, vol. 1 (11), ISSN 0960-894X, 579-80
- **RIDDLES PW ; BLAKELEY RL ; ZERNER B.** Reassessment of Ellman's reagent. *Methods Enzymol.,* 1983, vol. 91, 49-60